# EUROPEAN PATENT SPECIFICATION

(11) **EP 3 845 633 B1**
(45) Date of publication and mention of the grant of the patent: **15.04.2026**
(21) Application number: 20217729.1
(22) Date of filing: 29.12.2020
(51) Int. Cl.: C12N 1/20, C12N 11/16

(54) **A METHOD FOR MAKING SPATIALLY DEFINED AGGREGATES BY PRECISELY POSITIONING CELLS BASED ON ELECTROSTATIC INTERACTION**
VERFAHREN ZUR HERSTELLUNG RÄUMLICH DEFINIERTER AGGREGATE DURCH PRÄZISE POSITIONIERUNG VON ZELLEN AUF BASIS ELEKTROSTATISCHER WECHSELWIRKUNG
PROCÉDÉ DE FABRICATION D'AGRÉGATS SPATIALEMENT DÉFINIS EN POSITIONNANT PRÉCISÉMENT DES CELLULES BASÉES SUR UNE INTERACTION ÉLECTROSTATIQUE

(30) Priority: 31.12.2019 GB 201919467
(43) Date of publication of application: 07.07.2021
(73) Proprietor: Jozef Stefan Institute, 1000 Ljubljana (SI); Institut za metagenomiko in mikrobne tehnologije d.o.o., 1000 Ljubljana (SI)
(72) Inventor: LAPANJE, Ales, 1000 Ljubljana (SI); RIJAVEC, Tomaz, 1000 Ljubljana (SI)
(74) Representative: Patentni Biro AF d.o.o.

(56) References cited:
- GB-A- 2 431 472
- US-B1- 6 699 501
- VERDUZCO-LUQUE C. E. ET AL: "Construction of biofilms with defined internal architecture using dielectrophoresis and flocculation", BIOTECHNOLOGY AND BIOENGINEERING, vol. 83, no. 1, 5 July 2003 (2003-07-05), pages 39 - 44, XP002478960, ISSN: 0006-3592, DOI: 10.1002/BIT.10646
- TREWEEK G. P. ET AL: "The mechanism of E. coli aggregation by polyethyleneimine", JOURNAL OF COLLOID AND INTERFACE SCIENCE, vol. 60, no. 2, 15 June 1977 (1977-06-15), pages 258 - 273, XP024188910, ISSN: 0021-9797, [retrieved on 19770615], DOI: 10.1016/0021-9797(77)90286-7
- RYBKIN I. ET AL: "Thickness of Polyelectrolyte Layers of Separately Confined Bacteria Alters Key Physiological Parameters on a Single Cell Level", FRONTIERS IN BIOENGINEERING AND BIOTECHNOLOGY, vol. 7, 378, 4 December 2019 (2019-12-04), pages 1 - 10, XP055783329, DOI: 10.3389/fbioe.2019.00378
- FAKHRULLIN R. F. ET AL: ""Face-Lifting" and "Make-Up" for Microorganisms: Layer-by-Layer Polyelectrolyte Nanocoating", ACS NANO, vol. 6, no. 6, 21 May 2012 (2012-05-21), US, pages 4557 - 4564, XP055798781, ISSN: 1936-0851, DOI: 10.1021/nn301776y
- FAKHRULLIN R. F. ET AL: "Cyborg cells: functionalisation of living cells with polymers and nanomaterials", CHEMICAL SOCIETY REVIEWS, vol. 41, 16 April 2012 (2012-04-16), UK, pages 4189 - 4206, XP055798986, ISSN: 0306-0012, DOI: 10.1039/c2cs15264a
- FAKHRULLIN R. F. ET AL: "Live celloidosome structures based on the assembly of individual cells by colloid interactions", PHYSICAL CHEMISTRY CHEMICAL PHYSICS, vol. 12, no. 38, 14 October 2010 (2010-10-14), pages 11912 - 11922, XP055798793, ISSN: 1463-9076, DOI: 10.1039/c0cp00131g
- CHEN J. ET AL: "Cationic polyelectrolyte functionalized magnetic particles assisted highly sensitive pathogens detection in combination with polymerase chain reaction and capillary electrophoresis", JOURNAL OF CHROMATOGRAPHY B, vol. 991, 9 April 2015 (2015-04-09), pages 59 - 67, XP029230183, ISSN: 1570-0232, DOI: 10.1016/J.JCHROMB.2015.04.002

## Description

### Field of the Invention

The present invention relates to cell aggregation and particularly, although not exclusively, to microbial or bacterial cell aggregation.

### Background

Recently, microbiological research has indicated that the individual placement of particular cells is important for community functioning, since the microbes can transfer metabolites more efficiently, produce biofilms and develop particular niches based on the local physicochemical properties.

These insights are not important only from the academic perspective, but are also extremely important for biotechnological applications in different biotechnology fields such as food biotechnology, remediation, biofouling etc. For example, the topographical placement of individual cells in crude cheese influence the taste, structure, ripening as well as ageing of the cheese.

Aggregates are important in the biofertilisation of crops by allowing different strains to be used in a very small space. Aggregates formed from strains that can fix nitrogen, dissolve inorganic material to release nutrients, or produce protecting substances and prevent diseases, can improve the growth of the plant and increase production of plant biomass.

Aggregates are also important in the purification of waste water or tap water contaminated with pesticides, especially when purification involves aerobic and anaerobic steps. The surface of the aggregates enables aerobic activity of microorganisms, whereas the internal part of the aggregate promotes anaerobic respiration and fermentation. Use of aggregates enables better colonisation of the sand particles due to the easier retention of aggregates within the sand particles. Retention of the aggregates is increased as a result of the larger size of aggregates compared to the individual cells (mm sized aggregates compared to µm sized cells).

Recently, multi stage biotechnological fermentation have been replaced by one fermentation processes using co-cultures. The co-culture is hard to control when cells are in the suspended form due to different growth kinetics. Moreover, currently the co-cultures are mainly used in a process when it is necessary:
1. To induce production of secondary substances, here the induction relies on interspecies interaction or quorum sensing compounds acting in trans.
2. To scavenge oxygen for fermentation of carbon hydrates to short fatty acids such as acetate and lactate.
3. To involve complex, but cheap substrates (e.g. molasses, cellulose, lignocellulose), which can be used for the production of biofuels, acetate, lactate.
4. To produce complex secondary metabolites involving large metabolic pathways, for which genes cannot be easily cloned into the biotechnologically applicable host cells,
5. To directly transfer autotrophically produced carbohydrates to the heterotrophic cells.

These systems of co-culture are hard to optimize due to different growth dynamics of the strains and increased distances between cells when complex substrates are broken down to the level that is acceptable for use by producers of substances. Scavenging oxygen is hard to monitor within the whole culture and currently there are no easy ways to do this. Also the co-culture process is often separated into two steps, aerobic and microaerophylic/anaerobic. The direct combining of an autotrophic process with a heterotrophic process is currently only possible in suspensions or formed capsules of genetically modified autotrophic organisms which are capable of transmembrane exporting of carbohydrates. It is limited in efficiencies due to the large distances between the cells.

In contrast, aggregation enables close contact between cells and more intensive interactions. The interactions facilitate (*i*) exchanging growth substrates, secondary metabolites as well as quorum sensing molecules, (*ii*) scavenging oxygen due to the local increase of the oxygen consumption, enabling formation of anaerobic niches within the aggregates (e.g. natural cosms in the biologically water treatment processes).

Important factors in cell aggregation are the size of the aggregates, distribution of cells within the heterocellular aggregates (aggregate structure) and number of the aggregates. Size of the aggregate determines the consumption of nutrients and flux of gases within the layers of the aggregates, and also determines the distribution of the aggregated cells in the fluid bed, slow/quick sand filters, or other sorts of column type bioreactors. Size of the aggregate also determines the sedimentation of the biomass. Easier sedimentation also simplifies multiple reuse of the biomass in the fermenters.

The structure of the aggregates determines how cells are distributed within the aggregate. This is extremely important since the distribution of different types of cells determines the cascades of the biotechnological processes. Cells closer to the surface are involved in the earlier steps (e.g. degrading lignocellulose) than cells deeper within the aggregates which are involved in the later steps (e.g. fermentation and production of biofuels or acetate, lactates). Moreover, oxygen scavenging occurs in the most outer layers of the aggregates, enabling anaerobic processes to occur within the aggregates (e.g. nitrification and denitrification coupling of different microbial cells).

US 6,699,501 describes a process for encasing a biological or amphiphilic template in a capsule composed of consecutive layers of oppositely charged polyelectrolytes. The template can be disintegrated to leave the case intact. 2-40 layers can be deposited.

US 6,967,085 describes using polyelectrolytes of two different viscosities to flocculate cell material. These can be added simultaneously, sequentially, or as a pre-formed blend. One polyelectrolyte is cationic and the second polyelectrolyte is cationic or substantially non-ionic.

US 9,371,554 describes a method for harvesting recombinant protein by flocculating cells using cationic polyelectrolytes, allowing to settle, then removing supernatant.

IN 217966 B describes a process for artificially flocculating yeast particles by addition of cationic polymer material and a divalent metal ion.

Fakhrullin RF, Zamaleeva Al, Minullina RT, Konnova SA, Paunov VN., "Cyborg cells: functionalisation of living cells with polymers and nanomaterials", Chem Soc Rev., 2012, Jun 7; 41(11):4189-206 describes living cells with polyelectrolyte coatings, magnetic and metal nanoparticles or hard mineral shells. It describes methods for functionalisation of cells with polymers and nanoparticles and talks about cell viability and cell toxicity.

Fakhrullin RF, Lvov YM., ""Face-lifting" and "make-up" for microorganisms: layer-by-layer polyelectrolyte nanocoating", ACS Nano., 2012, Jun 26; 6(6):4557-64 describes sequential adsorption of oppositely charged polyelectrolytes onto living biological cells in mild aqueous conditions.

Wang SK, Wang F, Hu YR, Stiles AR, Guo C, Liu CZ., "Magnetic flocculant for high efficiency harvesting of microalgal cells", ACS Appl Mater Interfaces., 2014, Jan 8; 6(1):109-15 describes synthesis of a magnetic flocculent using iron oxide and cationic polyacrylamide. This was added to algal broth and used to harvest microalgal cells post-flocculation, with a magnet.

Verduzco-Luque CE et al., "Construction of biofilms with defined internal architecture using dielectrophoresis and flocculation", Biotechnology and Bioengineering, 2003, July 5; 83(1):39-44 describes an approach for the construction of biofilms with defined internal architecture using AC electrokinetics and flocculation. Artificial structured microbial consortia (ASMC) consisting of localized layered microcolonies of different cell types were formed by sequentially attracting different cell types to high field regions near microelectrodes using dielectrophoresis. Stabilization of the microbial consortia on the electrode surface was achieved by crosslinking the cells using the flocculant polyethyleneimine (PEI).

Rybkin I et al., "Thickness of Polyelectrolyte Layers of Separately Confined Bacteria Alters Key Physiological Parameters on a Single Cell Level", Frontiers in Bioengineering and Biotechnology, 2019, December 4; 7(378):1-10 describes a layer-by-layer method of deposition of a polyelectrolyte film a few nanometers in thickness to confine separated bacterial cells in permeable and physically durable shells. Prevention of aggregates was studied using PEI deposition on cells.

The present invention has been devised in light of the above considerations.

### Summary of the Invention

In contrast to the previous work, the present inventors have devised a method for topographically controlled aggregation, wherein cells of the same type or different type, such as different strains of bacterial cells, are layered in a way that controls the size, structure and number of aggregates as well as the potential spatial distribution of the different species of cells within the aggregates.

Current status of the knowledge in the field shows two perspectives (i) that separate bacterial cells can be modified using polyelectrolytes of high charge densities and (ii) the suspension of bacterial cells can be aggregated spontaneously and randomly. The invention provides a method for preparing spatially controlled aggregates comprising core cells, covered with shell cells, said core and shell cells having opposite surface potential.

Discussed herein are the surface potentials of cells. In general, cells naturally have a negative surface potential. Through surface modification, as explained herein, this can be switched to a positive surface potential, or enhanced to become a more negative surface potential. Cells with a negative surface potential are sometimes referred to herein as "negatively charged cells" for convenience. Similarly, cells with a positive surface potential are sometimes referred to herein as "positively charged cells" for convenience. This discussion of positive or negative refers to the effective surface potential of the cell, rather than an internal or intrinsic property of the cell itself.

The invention is set out in the appended set of claims.

In a first aspect, disclosed is a method for making an aggregation of cells, the method comprising the steps of (a) treating a first portion of cells with a positively charged polyelectrolyte to form a first portion of surface modified cells (i.e. first cells) and (b) mixing the first portion of surface modified cells with a second portion of cells (i.e. second cells), to form aggregations of cells each having a negative surface potential and each comprising a layer of cells on the surface of a surface modified cell.

In some embodiments the second portion of cells is not surface modified.

In some embodiments the second portion of cells comprises a second portion of surface modified cells formed by a method comprising the steps of (i) treating cells with a positively charged polyelectrolyte and then treating said cells with a negatively charged polyelectrolyte, to form the second portion of surface modified cells. These steps can modify the natural negative charge of the surface of the cell in order to promote the formation of aggregates.

In some embodiments the first portion of surface modified cells or the second portion of cells is treated with magnetic particles to coat their surface with such particles or embed such particles onto their surface, before the step of mixing the first portion of surface modified cells with the second portion of cells. This permits the aggregates to be separated easily using a magnet. In some embodiments the magnetic particles are paramagnetic. In some embodiments the magnetic particles are Fe₃O₄ particles.

In some embodiments each positively charged polyelectrolyte is individually selected from polyethyleneimine, polydiallyldimethylammonium chloride, poly(allylamine hydrochloride), chitosan, cationic starch and amino cellulose.

In some embodiments each negatively charged polyelectrolyte is individually selected from polystyrene sulfonate, polyacrylic acid, alginate or cellulose.

In some embodiments the first portion of cells and the second portion of cells are of different types. Using different types of cells enables the use of different cellular processes in the same small space.

In some embodiments each cell is a microbial cell, preferably a bacterial cell.

Another aspect that is disclosed is a method for making an aggregation of cells, the method comprising the step of treating an aggregation of cells with a negative surface potential, as made by the method of any preceding claim, with a portion of cells having a positive surface potential, to form an aggregation of cells each having a positive surface potential and each comprising a layer of cells having a positive surface potential, on the surface of a layer of the second portion of cells, on the surface of a surface modified cell. By this method a multi-layered cellular aggregate can be formed.

The present disclosure also relates to cell aggregates made by the methods described herein.

The disclosure includes the combination of the aspects and preferred features described except where such a combination is clearly impermissible or expressly avoided.

### Summary of the Figures

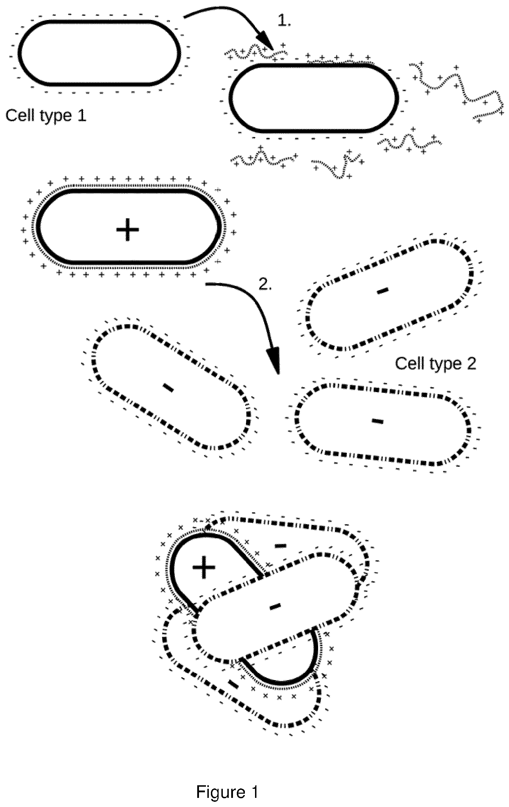

Embodiments and experiments illustrating the principles of the disclosure will now be discussed with reference to the accompanying figure 1, which shows the preparation of artificially constructed multicellular aggregates produced from two different types of bacterial cells. (1) The surface charge/potential of the first cell type is altered to be positive using a layer by layer approach. The altered surface charge of the cell enables its attachment to the negatively charged cells of the second cell type. (2) The appropriate procedure of mixing and ratio of positively to negatively charged cells results in formation of aggregates consisting of different combinations and number of cells.

### Detailed Description

Aspects and embodiments of the present disclosure will now be discussed with reference to the accompanying figures. Further aspects and embodiments will be apparent to those skilled in the art.

In the present disclosure, a first set or portion of cells is made to have a positive charge (that is, a positive surface potential). Those cells are then mixed with a second set or portion of cells, which have a negative charge (that is, a negative surface potential). When positive cells are added slowly to negative cells, the different charges/potentials mean that the negative cells agglomerate around the positive cell. If negative cells are added slowly to positive cells, the positive cells agglomerate around the negative cells.

The present disclosure describes techniques for controlling the surface potential/charge (both type, positive or negative, and magnitude) of cells, by use of suitably charged polyelectrolyte coatings for surface modification. Thus aggregates comprising the desired cells in the desired layering are possible.

Furthermore, the present disclosure describes techniques by which the cells can be multiply surface modified by addition of several polyelectrolytes. This enables the layers of the eventual aggregate (which will comprise those cells) to be engineered as desired.

Furthermore, the present disclosure describes techniques by which the aggregates themselves can be made to the desired size and layering.

The present disclosure also describes techniques for making the aggregates magnetic, thereby improving the separability and ultimately the yield of them.

It is noted that in all the procedures described herein, the temperature of the cells is suitably kept within the range of 0 to 37°C.

### Cell Types

Cells, for example microbial or bacterial cells, are well known. The present disclosure can be used with any types of cells, although it is particularly applicable to bacterial cells.

Cells generally have a naturally negative surface potential (naturally negatively charged). Suitably, the cells naturally have a surface potential of -100 to -10 mV, for example around -30 mV.

Examples of suitable cell types include bacterial cells. Any bacteria type can be used, provided that conditions for deposition of polyelectrolyte layers are determined by establishing the suitable stage of growth, surface pre-treatment, suitable centrifugation speed, and toxicity of different polyelectrolytes. Examples of suitable bacteria cells are *Lactococcus lactis, Pseudomonas spp., Escherichia coli,* and *Bacillus spp., Nitrobacter winogradskyi.*

In the present disclosure, a first set or portion of cells are made to have a positive charge (that is, a positive surface potential). Those cells are then mixed with a second set or portion of cells, of the same or different type, which have a negative charge (that is, a negative surface potential). Depending on the mixing conditions, the different charges/potentials mean that the negative cells agglomerate around the positive cells or the positive cells agglomerate around the negative cells.

The cells used in the first set or portion of cells and the second set of cells may be the same or different. For example, the sets or portions of cells may be of the same bacterial strain, or of different bacterial strains.

Thus, the present disclosure allows aggregation of a layer of bacterial strain A on/around bacterial strain B by suitable surface modification and mixing. Similarly, a layer of a further bacterial strain (the same or different) can be then layered on that, giving a multiple-layered structure with each layer comprising cells which may be the same as or different from the cells in any other layer of the aggregate.

### Cell Selection

Ideally, although not essentially, the cells used in the present disclosure can be selected such that they will have properties best suited to the aggregation procedure. For example, by selecting cells which are at an appropriate growth stage, the cells may have low electrophoretic softness (λ⁻¹) as well as a high surface charge (ZN).

Cells can be cultivated, taken from different growth stages (for example, late lag, mid exponential and early stationary) and measured for electrostatic mobility dependent on the ionic strength using solutions of monovalent ions (for example, NaCl solution from 0.02 to 0.12 M by 11 measurements with 10 fold increased concentrations of ions).

The obtained curves can then be used as a basis for the calculation of the λ⁻¹ and ZN using the Ohshima model.

Before the measurement, the cells may be washed by centrifugation. There may be 1-5 such washings, for example 3 washings. The centrifuge may be, for example, at 3000 g for 3 mins). The cells may then be resuspended, for example in a 0.00062M NaCl solution prepared in deionised water (18 MOhm), and dispersed using, for example, maximum vortexing of the suspension of cells for 10 s.

When the appropriate growth stage is determined (that is, by considering the λ⁻¹ and ZN at different growth stages and assessing which gives the ideal balance of λ⁻¹ and ZN), the bacterial cells for use in the invention can be prepared from a fresh culture.

A suitable protocol is that the overnight liquid cultures obtained from a single colony grown on solid medium are taken and 1 mL of this culture is transferred into a 100 mL fresh medium and incubated until reaching the optical density/ies corresponding to the previously determined appropriate growth stage.

A suitable such optical density may be, for example, an OD660nm of 1.0 to 1.5, for example 1.1 to 1.3, and suitably around 1.2.

### Surface modification - switching the surface potential of cells

In order to create cells with a positive charge (positive surface potential), the cell surfaces must be modified. In the present invention this is done by using a polyelectrolyte.

In order to change the negative surface potential of a cell to a positive one, a positively charged polyelectrolyte is used to treat the cells. It is attracted to the surface of the cell and forms a positively charged coating or layer; this renders the cell itself as having a positive surface potential (positively charged).

It will be appreciated that the same technique can be used to change the surface potential of a cell which has been surface modified to have a negative surface potential, as explained in more detail below.

For example, a suitable surface potential for such coated cells might be 10-100 mV, for example around 30 mV.

Various positively charged polyelectrolytes are suitable for use here. Depending on the target surface potential/charge, the positive polyelectrolyte can be selected suitably.

For example, the positive polyelectrolyte may be one with a high charge density of, for example, 1 chargeable moiety per 3 heavy atoms (for example polyethyleneimine), or one with a moderate charge density of, for example, 1 chargeable moiety per 10 heavy atoms (for example, chitosan), or one with a low charge density of, for example, 2 to 5 chargeable moieties per 100 heavy atoms (for example, cationic starch). As used herein, a heavy atom is one that is not hydrogen. Other examples of suitable positive polyelectrolytes are polydiallyldimethylammonium chloride and poly(allylamine hydrochloride).

A positively chargeable moiety is one which may be protonated (such as N, NH or NH2 in tertiary, secondary and primary amines respectively). A negatively chargeable moiety is one which may be deprotonated (such as SO3H or COOH). For example, a suitable positively chargeable moiety may have a pKa of 6.5 to 11 and a suitable negatively chargeable moiety has a pKa of 1 to 7.5.

The cells are coated with a layer of the positive electrolyte by, for example, mixing solutions thereof. Before mixing with the positive polyelectrolyte solution, the cells may be subjected to treatment to break up any existing flocculation. Such treatment may be, for example, by ultrasound. Suitable conditions for that are 80 kHz, 100% power, for 3 minutes.

A positive polyelectrolyte solution of 0.025 to 0.25% v/w at pH 7 (adjusted by using, for example, acid such as HCl) may suitably be added to a solution of the cells to be modified. That is, the amount of positive polyelectrolyte in the positive polyelectrolyte solution may be 0.025 to 0.25% w/v, suitably 0.06% to 0.25% w/v. The positive polyelectrolyte solution may be added to the cell solution at around 1:1 v/v.

The mixed solution can be left to stand for the coating to occur, for example at room temperature for 5 minutes.

After coating, any excess positive polyelectrolyte can be removed. This may be done by, for example, centrifugation (for example at 700 to 1300 g, chosen appropriately depending on the cells and polyelectrolyte present; the centrifugation step may be, for example, around 2-3 minutes). The obtained pellet is washed by removing the supernatant and slowly adding a new solution of appropriate ionic strength and pH to fill the whole centrifuge tube three times, being careful not to disturb the pellet. It will be appreciated by those skilled in the art that an appropriate ionic strength and pH may be selected to enable solubility of the polyelectrolyte and high survivability of the cells. This will vary depending on the types of cell and polyelectrolyte.

An appropriate positive polyelectrolyte and its concentration for mixing can be determined by the minimal inhibitory method using different amounts of polyelectrolytes and a standard amount of cells (around 100 CFU in 200 µl of media). By this method, cells are firstly washed and the number of cells is determined via optical density. This suspension of cells is added to the polyelectrolyte solutions in 1:1 ratio (v/v). The suspension is washed via centrifugation method to remove all the polyelectrolytes from the solutions and then the zeta potential is measured using electrophoretic light scattering (ELS) method. Polyelectrolyte solution is diluted two fold from 0.5 to 0.003. At each dilution the cells are added to the polyelectrolyte solution and the washing procedure repeated and the zeta potential measured. The decreasing power of surface charge is correlated with the amount of charged polyelectrolytes attached on the bacterial cells. Relevant concentrations are based on the determined electromobilities per cell:polyelectrolyte concentrations, which are suitably above 5mV.

The concentration window of polyelectrolytes and charge densities is accordingly determined within the limits of the concentrations allowing the cells to survive while still being sufficient to change the surface potential to be positive.

### Surface modification - enhancing the charge of cells

While cells generally have a naturally negative charge (surface potential), it may be advantageous to increase it, that is, make it more negative. For example, this might enhance the strength of interaction with the positively charged cells, leading to better or stronger aggregation.

Where this is desired, positively charged cells (for example, made as explained above) can be treated with a negatively charged polyelectrolyte in order to apply a layer of negatively charged polyelectrolyte to them. This negative polyelectrolyte can impart an effective negative charge (negative surface potential) on the cell which is 'greater' (more negative) than that which it naturally had.

Suitable negative polyelectrolytes include, for example, polystyrene sulfonate, polyacrylic acid, aliginic acid and cellulose.

As with the positive polyelectrolyte, the negative polyelectrolyte can be layered onto the positive cells by mixing a solution of it with a solution of positively charged cells (for example as made above).

The pH of the negative polyelectrolyte solution may be adjusted, for example to about 7, using an alkali such as NaOH.

A negative polyelectrolyte solution of 0.025 to 0.25% w/v at pH 7 may suitably be added to a solution of the cells to be modified. That is, the amount of negative polyelectrolyte in the negative polyelectrolyte solution may be 0.025 to 0.25% w/v.

The negative polyelectrolyte solution may be added to the (positive) cell solution at around 1:1 v/v.

The mixed solution can be left to stand for the coating to occur, for example at room temperature for 5 minutes.

After coating, any excess negative polyelectrolyte can be removed. This may be done by, for example, centrifugation (for example at 1300 to 2200 g, chosen appropriately depending on the cells and polyelectrolyte present; the centrifugation may be for, for example, around 2-3 minutes). A higher gravitational force is used than for the centrifugation of the positive cells mentioned above.

The obtained pellet may then be washed to further remove polyelectrolyte by removing the supernatant and slowly adding a new solution of appropriate ionic strength and pH three times, being careful not to disturb the pellet.

### Forming Aggregates

In the present disclosure, aggregates are generally formed by mixing positively charged (positive surface potential) cells with negatively charged (negative surface potential) cells. These may be made by the methods explained above. The negatively charged (negative surface potential) cells may be natural cells, that is, cells with no alteration of their surface potential. Alternatively, they may be cells which have a positively charged polyelectrolyte surface modification and an external negative polyelectrolyte surface modification.

The mixing of the positively charged cells and the negatively charged cells may be done in any manner. For example, a solution of the positively charged cells may be added to a solution of the negatively charged cells. Or, for example, a solution of the negatively charged cells may be added to a solution of the positively charged cells. Or, for example, solutions of the positively charged cells and the negatively charged cells may be added simultaneously to a receptacle. The core of the aggregate is formed by the cell type which is present in the lower amount. Suitable ratios of core to coating cell type are 1:8 to 1.4:100.

For example, the negatively charged cells may be placed in a 5 ml microcentrifuge tube. The mixer may be mounted such that the mixing head is placed at the bottom of the conical shaped part of the 5 ml microcentrifuge tube. The mixer may be set at, for example, 2000 to 8000 rpm, for example 3000 to 6000 rpm, suitably 5000 rpm. The positively charged cells can then be added using a syringe pump at, for example, 50 to 500 µl h⁻¹, for example 100 to 300 µl h⁻¹, for example about 200 µl h⁻¹, Depending on the amount to be added, such addition may continue for, for example, 5 to 60 minutes, for example 10 to 40 minutes, suitably about 20 min.

The mixing of these solutions forms a suspension of aggregated cells and solitary cells. The amount of aggregation can be controlled by adjustment of the concentration of each cell type, the flow rate of the cells that are added to the suspension of oppositely charged cells, mixing/stirring speed (to ensure a well distributed suspension of cells), amount of added oppositely charged cells to suspension of cell, and ionic strength of cell surface. This allows for control of the number of aggregates in the solution.

Aggregation can be monitored using subsamples by, for example, light microscopy, dynamic light scattering size distribution or flow cytometry procedures. Light microscopy involves finding aggregates at lower magnifications and then determining the distribution of cells within the aggregates. The cells, suitably the cells forming the core of the aggregates, can be fluorescently labelled prior to aggregation, and distribution can be monitored by fluorescent microscopy. Dynamic light scattering size distribution exploits the thermal random movement wherein larger particles move further in one direction due to gravitation force. Therefore, the relative number of aggregates of a determined size can be estimated. In flow cytometry procedures one of the cells must be labelled fluorescently. Cells and aggregates of cells pass through a beam and scatter light. The side and forward scatter can show the size of the aggregate and the fluorescent signal determines at which size the fluorescent cores are formed. Accordingly, number and size of aggregates, and number of non-aggregated cells can be determined.

### Layering

The aggregates themselves comprise a core or centre of a cell or small flocculation of cells having one surface potential (positive, by surface modification, or negative, naturally or by surface modification), with a layer or coating of the oppositely charged/opposite surface potential cells (negative or positive) upon it.

That layer or coating may itself have a further layer or coating of cells upon it, having the opposite surface potential (positive or negative).

This layering of cells, and accordingly the size of the eventual product aggregates, can be controlled by using sequential alternating cell coating steps.

For example, a series involving coating natural/uncoated cells with positively charged cells; followed by coating with negatively charged cells; followed by coating with positively charged cells leads to aggregates which have a negative core and then three layers on top. In this way the species of cell that composes each layer can be controlled, enabling a combination of different cell layers to form multistep biocatalytic aggregates. The greater the number of layers, the more biotechnological processes can be combined in a very small space to work simultaneously, without the need for separated reactors or conditions. A suitable number of layers is 2 to 20.

In the above discussions, it is explained how the surface potential of a cell can be switched, from negative to positive, and indeed switched back to negative by addition of a further polyelectrolyte layer.

It is envisaged that repeated addition of layers of polyelectrolytes, to form layers of alternating charge on a single cell, by the procedures set out above, may be possible. This can control the individual size of the surface modified cells, which thus affects the thickness or size of the layer(s) in the aggregate formed using those cells.

By suitable layering of polyelectrolytes on cells, followed by suitable layering of the cells to form aggregates, the present techniques allow for great control of the positioning of cells within an aggregate, their concentration therewithin, the size of those aggregates and the thicknesses of the cell layers.

### Separating Aggregates

Once aggregates have been formed, for example within a solution, they can be removed or separated from it.

Suitable techniques include size exclusion, differential centrifugation in gradients, filtration, dielectrophoretic separation or dielectric separation due to the different charge distribution in aggregates than in individual cells and bulk density properties of aggregates. Cells can be also distinguished from aggregates via flow cytometry, and so the aggregates can be separated by flow sorters.

To increase the ease of separability, and the yield of such a procedure, the inventors have found that it is advantageous to make the aggregates themselves magnetic. This permits separation using a magnet to draw the aggregates to it, allowing for easier removal of the rest of the solution (for example) and more effective washing of the remainder (because the aggregates are magnetically held).

This can be achieved by, for example, fully or partially coating certain cells with a magnetic material. For example, the positively charged cells, or the negatively charged cells, may be coated with particles of a magnetic material. In some embodiments the positively charged particles are coated fully or partially with the magnetic material.

Magnetic particles are well known. Suitable particles include, for example, paramagnetic particles such as Fe₃O₄ particles.

The particles may be nanoparticles, for example, having a particle diameter of 5 nm or more and 1000 nm or less. The particle diameter may be measured by dynamic light scattering or electron microscopy.

Such a layer can be added by mixing a solution of the magnetic particles with a solution of the cells to be coated.

For example, to cells coated with a positive polyelectrolyte, a suspension containing Fe3O4 can be added. The suspension may contain, for example, 0.25% magnetite. This is added in an amount of around 1:1 v/v. The Fe3O4 particles then coat onto the positively charged cells.

To separate out cells with a negative surface potential, the magnetites can be pretreated to change the surface charge to a positive one and used in the same way as above. A permanent magnetic field can then be used to hold the coated cells while the uncoated cells are washed out.

After an initial wash, the remainder (magnetic cells plus unattached Fe₃O₄) can be suspended in solution, for example a 0.9% NaCl solution in water. This can then be centrifuged (at, for example, 1000-2000 g for 2-3 minutes) to remove the unattached Fe3O4. The thus prepared cells, with a Fe₃O₄ coating, can be recoated by the procedures set out above for changing or enhancing the charge of cells, to give the desired surface charge/potential.

For example, the prepared cells can be suspended in 0.9% NaCl solution in water and a solution of polyelectrolyte added to them. Where the cells were positively charged before the magnetic coating was added, a positive polyelectrolyte can be used to re-impart a positive surface charge/potential.

After that, the magnetic cells can be isolated by removal of the unused polyelectrolyte by centrifugation and washing (for example 3-4 times, using 0.9% NaCl solution in water).

The magnetic cells can then be used for formation of aggregates, or indeed for addition of further polyelectrolyte layers, as described herein.

When aggregates are made using such magnetic cells, the separation of the aggregates is simplified and improved. The suspension, comprising aggregates as well as solitary coating cells, which are present in excess, can be exposed to a permanent magnet (for example, 1T). The solution of unattached aggregates can then be removed. The unattached cells and aggregates can gradually be washed out, with the aggregates being kept 'wet' (or at least not dry) by addition of NaCl solution in deionised water at a maximum of 1.5% NaCl. Two or more such washes can remove the remaining solitary cells.

The permanent magnet can then be removed, and the aggregates resuspended, for example in NaCl solution at maximum 1.2% NaCl in deionised water. The aggregates are then ready for further addition of cell layers as described herein, if wanted.

The prepared aggregates can be then physically stabilised by encapsulation either using additional polyelectrolyte coating over the aggregates, covalent crosslinking or using matrix stabilization such as alginate, polyacrylamide beads or other gels. Mechanical encapsulation enables the aggregates to maintain stability when cells start to divide.

The features disclosed in the foregoing description, or in the following claims, or in the accompanying drawings, expressed in their specific forms or in terms of a means for performing the disclosed function, or a method or process for obtaining the disclosed results, as appropriate, may, separately, or in any combination of such features, be utilised for realising the invention in diverse forms thereof.

For the avoidance of any doubt, any theoretical explanations provided herein are provided for the purposes of improving the understanding of a reader. The inventors do not wish to be bound by any of these theoretical explanations.

Any section headings used herein are for organizational purposes only and are not to be construed as limiting the subject matter described.

Throughout this specification, including the claims which follow, unless the context requires otherwise, the word "comprise" and "include", and variations such as "comprises", "comprising", and "including" will be understood to imply the inclusion of a stated integer or step or group of integers or steps but not the exclusion of any other integer or step or group of integers or steps.

It must be noted that, as used in the specification and the appended claims, the singular forms "a," "an," and "the" include plural referents unless the context clearly dictates otherwise. Ranges may be expressed herein as from "about" one particular value, and/or to "about" another particular value. When such a range is expressed, another embodiment includes from the one particular value and/or to the other particular value. Similarly, when values are expressed as approximations, by the use of the antecedent "about," it will be understood that the particular value forms another embodiment. The term "about" in relation to a numerical value is optional and means for example +/- 10%.

### Examples

### EXAMPLE 1

### (i) Preparation of cells

The goal of this procedure is to define appropriate growth stage of the cell to achieve the lowest electrophoretic softness (λ⁻¹) as well as the highest surface charge (ZN).

Nonmotile cells of *Escherichia coli* top 10 strain (F- mcrA Δ(mrr-hsdRMS-mcrBC) Φ80lacZΔM15 ΔlacX74 recA1 araD139 Δ(ara leu) 7697 galU galK rpsL (StrR) endA1 nupG) were transformed with pRSET- emGFP plasmid (Thermo Fisher Scientific Corp.) using standard electroporation procedures. The plasmid contained T7 promoter regions upstream of the emGFP reporter gene and ApR cassette. Since cells are deficient in T7 polymerase, the GFP is transcribed due only to promotor leakage. The transformants were cultivated at 37 °C on nutrient agar (NA) plates (Sigma-Aldrich) supplemented with ampicillin (100 µg/ml, Sigma-Aldrich) - NAamp.

For efficient polyelectrolyte deposition, the electrostatic properties of the surface of bacterial cell in different growth stages were determined. The charge densities (ZN) and the electrostatic softness parameter (λ⁻¹) of bacterial cells were determined from a nonlinear regression analysis of ionic strength dependant electrophoretic mobilities (ISDEM) of bacterial cells by using Ohshima's soft particle equation as a model.

To obtain the ISDEM, the cultures were washed 3 times by centrifugation (3000g for 3min), pouring out of the supernatant, and resuspension in 0.00062M NaCl solution prepared from deionised water (18MOhm), being careful not to disturb the pellet. Then 100 µl of washed culture was mixed with 900 µl of 0.00062 M NaCl solution and the cells were properly dispersed using maximum vortexing of the suspension of cells for 10s. The electrophoretic mobilities of bacterial cells were measured using an ELS device (Zetasizer Nano, Malvern, USA). The ionic strength of the suspension of bacterial cells within the measuring cuvette was automatically altered with titration using an MPT-2 titrator. Measurements were made within the linear gradient of ionic strengths of NaCl from 0.00062 M to 0.11 M in 12 steps of 0.0091 M per step by addition of the 0.155 M solution of NaCl. The data was obtained from 3 experimental replicates where each of the measurements was acquired from the accumulated values of 70 separated ELS values. In all ELS measurements the polarities of electrodes were fixed, with an automatically adjustable voltage. The approximate values of ionic strengths were obtained from the titrator and the exact values for the cell suspensions within cuvettes were determined on a basis of conductivity values obtained in each ELS measurement. The ionic strengths in the ELS measurements were calculated on a basis of the standard curve values obtained from the measurement of the conductivities of different concentrations of NaCl solutions in water.

When the appropriate growth stage is determined the bacterial cells must be prepared from the fresh culture following the protocol: from the overnight liquid cultures obtained from a single colony grown on solid medium 1 mL of this culture is transferred into the 100 mL fresh medium and incubated with shaking at 37 °C and 150 rpm until reaching the optical densities corresponding to the previously determined appropriate growth stage.

*E. coli* cells were grown at 37 °C by shaking at 150 rpm until OD660 = 0.2 was reached. Cells were concentrated by centrifugation of 50 mL of the culture at 5000 g for 6 min. To wash out residues from the medium the pellet was washed three times by resuspension in 30 mL of 0.9% NaCl solution and centrifugation of the suspension at 3000 g for 3 min.

### (ii) Modification of surface potential of cells

In this example *E. coli* cells are used. To make the surface potential of the cells positive, poly(ethyleneimine) PEs (PEI, MW=750,000 g/mol) was used. To make the surface potential of the cell negative, charged sodium poly(styrene sulfonate) (PSS, MW = 70,000 g/mol) was used.

The solutions of PEs in Milli-Q (ultrapure water type 1) water (2.5 mg/ml, pH 7 adjusted by NaOH or HCl) were prepared by solubilizing PEs, initially by stirring and then by the sonication (35 kHz, 100W) for 15 min. In experiments using labeled PEI with tetramethylrhodamine isothiocyanate (TRITC), the PEI was labeled using NHS ester labeling of amino biomolecules. Briefly, the TRITC (11 mg in 11 mL DMSO solution) was added to a PEI solution (2.5 mg/ml in 20 mL of water) in a 50 mL tube and stirred at room temperature for 4 h. To remove the residual dye from the solution, after labeling the solution was dialyzed for 3 days using a dialysis tube (Orange Scientific) with nominal molecular weight limits between 12 and 14 kDa.

Before the deposition of the polyelectrolyte on the surface of cells, cells were exposed to the ultrasound at 80 kHz, 100% power, for 3 minutes. The polyelectrolyte was deposited on a layer by layer basis.

To prevent formation of aggregates of the cells, the parameters for efficient single cell PE deposition was determined. Polyethyleneimine (PEI) was deposited on the cells by adding a 0.25% solution of PEI at pH 7 (adjusted with HCl) in Milli-Q water to the washed cells (OD660 1.2) in a 1:1 v/v ratio. This suspension was stirred at room temperature for 5 min. Unattached PEI was removed from the suspension by centrifugation at 900 g for 2 min. The obtained pellet was washed twice by gentle addition of 1 ml of 0.9% NaCl over the pellet taking care to avoid disturbing it. After washing, the PEI covered cells were resuspended in 0.9% NaCl solution.

A PSS layer was deposited on the PEI covered suspension using a 0.25% w/v solution in Milli-Q water of PSS at pH 7 (adjusted with NaOH) in a 1:1 v/v ratio using the same procedure as above, except that 3 min centrifugation at 1500 g was used to obtain a sufficiently firm pellet to permit washing by pipetting.

By repetition of these two steps, four such layers were deposited on the surface of bacterial cells while keeping their aggregation, as monitored by microscopy, low.

Such prepared cells were then exposed to an ultrasound waterbath at 80 kHz, using 100% power, for 3 minutes.

### (iii) Aggregation of cells

The negatively charged cells with four layers of polyelectrolyte, prepared as above, were put into a 5 ml microcentrifuge tube. The mixer was mounted such that the mixing head was placed at the narrow end of the conical shaped part of the 5 ml microcentrifuge tube. The mixer was set at 5000 rpm. The positively charged *E. coli* cells with one polyelectrolyte layer, prepared as described in ii) above, were slowly added using a syringe pump at 200µl h⁻¹ for 20 min. A suspension of aggregated cells and solitary cells was formed.

### (iv) Extraction of aggregates

For extraction of aggregates using a magnet, *E. coli* cells with one positive polyelectrolyte layer were prepared as above and modified prior to aggregation. A suspension of 10-20 nm large citrate stabilized magnetite particles were added to the *E*. *coli* cells. Over that layer a negative layer was deposited and then a positive layer was deposited using the same procedure as ii) above. Aggregation was carried out as described in iii).

The suspension comprising aggregated as well as solitary cells was exposed to a strong permanent magnet (1 T), and left for 10 minutes under such exposure. This separated the magnetic aggregated cells. The solution of unattached aggregates was then taken out.

To wash out the remaining solitary cells from the separated aggregates, washing with 1.5% NaCl solution in water was conducted three times.

The tube was then removed from the permanent magnet and the remaining separated aggregates were resuspended in 100 µl NaCl solution at 1.2% NaCl in deionised water.

## Claims

1. A method for making spatially defined aggregates by precisely positioning cells based on electrostatic interaction, the method comprising the steps of:
a. surface modification of a first portion of cells to obtain cells with a positive surface potential i.e. first cells, said modification comprising:
- growing cells in a suitable growth medium to a suitable OD, washed and optionally treated to break any existing multicellular structures caused by spontaneous flocculation, coagulation or aggregation of cells,
- treatment of suspension of dispersed cells obtained in the previous step with a 0.025 to 0.25% w/v solution of a positively charged polyelectrolyte to form a first portion of surface modified cells; and removing excess polyelectrolyte, not associated with cells, out from the suspension,
b. forming aggregates of cells by mixing the first portion of surface modified cells with a second portion of cells with a negative surface potential i.e. second cells to form aggregates comprised of first cells as core cells covered with a layer of second cells as shell cells, said aggregates having a negative surface potential, or aggregates comprised of second cells as core cells covered with a layer of first cells as shell cells these aggregates having a positive surface potential, and wherein mixing in step b) comprises the following steps:
- placing first or second cells in a receptacle, which is a 5 ml microcentrifuge tube,
- mixing with a mixer set to 2000 to 8000 rpm, for example 3000 to 6000 rpm, suitably 5000 rpm,
- adding the cells with opposite surface potential to the receptacle, preferably using a syringe pump at 50 to 500 µl h-1, for example 100 to 300 µl h-1, for example about 200 µl h-1, to obtain a mixture of cells, and
- mixing of the mixture of cells for 5 to 60 minutes, for example 10 to 40 minutes, suitably about 20 min.

2. A method according to claim 1, wherein the second portion of cells are natural cells without modified surface or the cells have a modified surface with negative surface potential and were obtained by a method comprising the steps of:
i. growing cells in a suitable growth medium to a suitable OD, the cells being washed and optionally treated to break any existing multicellular structures caused by spontaneous flocculation, coagulation or aggregation of cells,
ii. treating cells with a 0.025 to 0.25% w/v solution of a positively charged polyelectrolyte;
iii. removing excess polyelectrolyte, and then
iv. treating obtained cells with a 0.025 to 0.25% w/v solution of a negatively charged polyelectrolyte, to form the second portion of surface modified cells
v. and finally removing excess of negatively charged polyelectrolyte.

3. A method according to claim 1 or 2, wherein the first portion of cells obtained in step a) is further treated with a 0.025 to 0.25% w/v solution of a negatively charged polyelectrolyte and afterwards with a 0.025 to 0.25% w/v solution of a positively charged polyelectrolyte, wherein the number of further treatment steps is arbitrary.

4. A method according to any of claims 1 to 3, wherein the surface modified first cells or second cells prior to step b have several layers of polyelectrolyte coatings, for example a first layer with positively charged polyelectrolyte, a second layer with a negatively charged polyelectrolyte, a third layer of positively charged polyelectrolyte and optional further layers.

5. A method according to any one of claims 1 to 4, wherein the process of aggregation is controlled by adjustment of the concentration of core and shell cells, the flow rate of the cells that are added to the suspension of oppositely charged cells, mixing/stirring speed, amount of added oppositely charged cells to suspension of cell, and ionic strength of the suspension of cells.

6. A method according to any one of claims 1 to 5, wherein the ratio of core cells to shell cells is 1:8 to 1.4:100.

7. A method according to any one of claims 1 to 6, wherein the first portion of surface modified cells or the second portion of cells is treated with magnetic particles to coat their surface with such particles or embed such particles onto their surface for enabling separation and possible enrichment of formed aggregates, wherein treatment with magnetic particles is performed before the step of mixing the first portion of surface modified cells with the second portion of cells.

8. A method according to claim 7, wherein the magnetic particles are paramagnetic.

9. A method according to claim 8, wherein the magnetic particles are Fe₃O₄ particles.

10. A method according to any of the preceding claims, wherein the obtained aggregates in step b) are further treated with the first portion of cells having a positive surface potential serving as third cells, to form aggregations of first cells covered with a layer of second cells further covered with a layer of third cells, the so obtained aggregations having a positive surface potential, or aggregates comprised of second cells covered with a layer of first cells are treated with the second portion of cells having a negative surface potential, so that the obtained aggregates have a negative surface potential, wherein the number of further layers of cells with alternating surface potentials is arbitrary.

11. A method according to any preceding claim, wherein each positively charged polyelectrolyte is individually selected from polyethyleneimine, polydiallyldimethylammonium chloride, poly(allylamine hydrochloride), chitosan, cationic starch and amino cellulose.

12. A method according to any preceding claim, wherein each negatively charged polyelectrolyte is individually selected from polystyrene sulfonate, polyacrylic acid, alginate or cellulose.

13. A method according to any preceding claim, wherein the first portion of cells and the second portion of cells and the third or any further portion of cells are of different types.

14. A method according to any preceding claim, wherein each cell is a microbial cell, preferably a bacterial cell.

## Patentansprüche

1. Verfahren zur Herstellung räumlich definierter Aggregate durch präzise Positionierung von Zellen auf Basis elektrostatischer Wechselwirkung, wobei das Verfahren die folgenden Schritte umfasst:
a. Oberflächenmodifizierung eines ersten Abschnitts von Zellen, um Zellen mit positivem Oberflächenpotential zu erhalten, d. h. erste Zellen, wobei die Modifizierung Folgendes umfasst:
- Züchten von Zellen in einem geeigneten Wachstumsmedium bis zu einer geeigneten optischen Dichte, gewaschen und gegebenenfalls behandelt, um vorhandene, durch spontane Ausflockung, Koagulation oder Aggregation entstandene multizelluläre Strukturen aufzubrechen,
- Behandlung der im vorherigen Schritt erhaltenen Suspension dispergierter Zellen mit einer Lösung von 0,025 bis 0,25 Gew.-% eines positiv geladenen Polyelektrolyten, um einen ersten Abschnitt oberflächenmodifizierter Zellen zu bilden; und Entfernen von überschüssigem, nicht an Zellen gebundenem Polyelektrolyt aus der Suspension,
b. Bilden von Zellaggregaten durch Mischen des ersten Abschnitts oberflächenmodifizierter Zellen mit einem zweiten Abschnitt von Zellen mit negativem Oberflächenpotential, d. h. zweiten Zellen, um Aggregate auszubilden, die aus ersten Zellen als Kernzellen, die mit einer Schicht zweiter Zellen als Hüllzellen bedeckt sind, bestehen, wobei die Aggregate ein negatives Oberflächenpotential aufweisen, oder Aggregate, die aus zweiten Zellen als Kernzellen, die mit einer Schicht erster Zellen als Hüllzellen bedeckt sind, bestehen, wobei diese Aggregate ein positives Oberflächenpotential aufweisen und wobei das Mischen in Schritt b) die folgenden Schritte umfasst:
- Platzieren erster oder zweiter Zellen in einem Gefäß, das ein 5-ml-Mikrozentrifugenröhrchen ist,
- Mischen mit einem Mischerset bei 2000 bis 8000 U/min, beispielsweise 3000 bis 6000 U/min, vorzugsweise 5000 U/min,
- Zugeben der Zellen mit entgegengesetztem Oberflächenpotential in das Gefäß, vorzugsweise unter Verwendung einer Spritzenpumpe mit 50 bis 500 µl/h, beispielsweise 100 bis 300 µl/h, beispielsweise etwa 200 µl/h, um eine Zellmischung zu erhalten, und
- Mischen der Zellmischung für 5 bis 60 Minuten, beispielsweise 10 bis 40 Minuten, vorzugsweise etwa 20 Minuten.

2. Verfahren nach Anspruch 1, wobei es sich bei dem zweiten Abschnitt der Zellen um natürliche Zellen ohne modifizierte Oberfläche oder um Zellen mit einer modifizierten Oberfläche mit negativem Oberflächenpotential handelt, die durch ein Verfahren erhalten wurden, das die folgenden Schritte umfasst:
i. Züchten von Zellen in einem geeigneten Wachstumsmedium bis zu einer geeigneten optischen Dichte, wobei die Zellen gewaschen und gegebenenfalls behandelt werden, um vorhandene, durch spontane Ausflockung, Koagulation oder Aggregation entstandene multizelluläre Strukturen aufzubrechen,
ii. Behandeln der Zellen mit einer Lösung von 0,025 bis 0,25 Gew.-% eines positiv geladenen Polyelektrolyten;
iii. Entfernen überschüssigen Polyelektrolyts und anschließend
iv. Behandeln der mit einer Lösung von 0,025 bis 0,25 Gew.-% eines positiv geladenen Polyelektrolyten erhaltenen Zellen, um den zweiten Abschnitt oberflächenmodifizierter Zellen zu bilden,
v. und schließlich Entfernen des Überschusses von negativ geladenem Polyelektrolyt.

3. Verfahren nach Anspruch 1 oder 2, wobei der erste Abschnitt der in Schritt a) erhaltenen Zellen weiterhin mit einer Lösung von 0,025 bis 0,25 Gew.-% eines negativ geladenen Polyelektrolyten und danach mit einer Lösung von 0,025 bis 0,25 Gew.-% eines positiv geladenen Polyelektrolyten behandelt wird, wobei die Anzahl der weiteren Behandlungsschritte beliebig ist.

4. Verfahren nach einem der Ansprüche 1 bis 3, wobei die oberflächenmodifizierten ersten Zellen oder zweiten Zellen vor Schritt b) mehrere Schichten von Polyelektrolytbeschichtungen aufweisen, beispielsweise eine erste Schicht mit positiv geladenem Polyelektrolyt, eine zweite Schicht mit negativ geladenem Polyelektrolyt, eine dritte Schicht mit positiv geladenem Polyelektrolyt, und optional weitere Schichten.

5. Verfahren nach einem der Ansprüche 1 bis 4, wobei der Aggregationsprozess durch Anpassung der Konzentration der Kern- und Hüllzellen, der Durchflussrate der der Suspension mit entgegengesetzt geladenen Zellen zugegebenen Zellen, der Misch-/Rührgeschwindigkeit, der Menge der der Zellsuspension zugegebenen entgegengesetzt geladenen Zellen und der Ionenstärke der Zellsuspension gesteuert wird.

6. Verfahren nach einem der Ansprüche 1 bis 5, wobei das Verhältnis von Kernzellen zu Hüllzellen 1:8 bis 1,4:100 beträgt.

7. Verfahren nach einem der Ansprüche 1 bis 6, wobei der erste Abschnitt oberflächenmodifizierter Zellen oder der zweite Abschnitt von Zellen mit magnetischen Partikeln behandelt wird, um deren Oberfläche mit solchen Partikeln zu beschichten oder solche Partikel auf ihre Oberfläche einzubetten, um die Trennung und mögliche Anreicherung gebildeter Aggregate zu ermöglichen, wobei die Behandlung mit magnetischen Partikeln vor dem Schritt des Mischens des ersten Abschnitts oberflächenmodifizierter Zellen mit dem zweiten Abschnitt von Zellen durchgeführt wird.

8. Verfahren nach Anspruch 7, wobei die magnetischen Partikel paramagnetisch sind.

9. Verfahren nach Anspruch 8, wobei die magnetischen Partikel Fe₃O₄-Partikel sind.

10. Verfahren nach einem der vorstehenden Ansprüche, wobei die in Schritt b) erhaltenen Aggregate weiterhin mit dem ersten Abschnitt von Zellen mit positivem Oberflächenpotential, die als dritte Zellen dienen, behandelt werden, um Aggregate aus ersten Zellen zu bilden, die mit einer Schicht zweiter Zellen bedeckt sind, die wiederum mit einer Schicht dritter Zellen bedeckt sind, wobei die so erhaltenen Aggregate ein positives Oberflächenpotential aufweisen, oder die Aggregate, die aus zweiten Zellen bestehen, die mit einer Schicht erster Zellen bedeckt sind, mit dem zweiten Abschnitt von Zellen mit negativem Oberflächenpotential behandelt werden, sodass die erhaltenen Aggregate ein negatives Oberflächenpotential aufweisen, wobei die Anzahl der weiteren Schichten von Zellen mit alternierenden Oberflächenpotentialen beliebig ist.

11. Verfahren nach einem der vorstehenden Ansprüche, wobei jedes positiv geladene Polyelektrolyt einzeln ausgewählt ist aus Polyethylenimin, Polydiallyldimethylammoniumchlorid, Poly(allylaminhydrochlorid), Chitosan, kationischer Stärke und Aminocellulose.

12. Verfahren nach einem der vorstehenden Ansprüche, wobei jedes negativ geladene Polyelektrolyt einzeln aus Polystyrolsulfonat, Polyacrylsäure, Alginat oder Cellulose ausgewählt ist.

13. Verfahren nach einem der vorstehenden Ansprüche, wobei der erste Abschnitt der Zellen und der zweite Abschnitt der Zellen und der dritte oder ein weiterer Abschnitt der Zellen von unterschiedlicher Art sind.

14. Verfahren nach einem der vorstehenden Ansprüche, wobei jede Zelle eine mikrobielle Zelle ist, vorzugsweise eine bakterielle Zelle.

## Revendications

1. Procédé de fabrication d'agrégats spatialement définis en positionnant précisément des cellules grâce à une interaction électrostatique, le procédé comprenant les étapes de :
a. modification de la surface d'une première série de cellules afin d'obtenir des cellules au potentiel de surface positif, qu'on appellera premières cellules, ladite modification comprenant :
- la culture de cellules dans un milieu de croissance approprié jusqu'à l'OD approprié, qui seront lavées et facultativement traitées pour briser d'éventuelles structures multicellulaires causées par la floculation spontanée, la coagulation ou l'agrégation de cellules,
- le traitement de la suspension de cellules dispersées obtenue à l'étape précédente avec une solution de polyélectrolyte de charge positive et de 0,025 à 0,25% p/v pour former une première série de cellules à la surface modifiée ; et le retrait du polyélectrolyte en excès, non associé aux cellules, de la suspension,
b. la formation d'agrégats de cellules en mélangeant la première série de cellules à surface modifiée avec une seconde série de cellules à potentiel de surface négatif, qu'on appellera cellules secondes, pour former des agrégats composés de cellules premières faisant office de cellules de noyau, recouvertes d'une couche de cellules secondes faisant office de cellules d'enveloppe, lesdits agrégats ayant un potentiel de surface négatif, ou des agrégats composés de cellules secondes comme cellules de noyau recouvertes d'une couche de cellules premières comme cellules d'enveloppe, ces agrégats ayant un potentiel de surface positif, et où le mélange de l'étape b) comprend les étapes suivantes :
- le placement des cellules premières ou secondes dans un récipient, qui est un tube de microcentrifugation de 5 ml,
- le mélange avec un mélangeur réglé sur 2000 à 8000 tr/min, par exemple 3000 à 6000 tr/min, idéalement 5000 tr/min,
- l'ajout des cellules ayant un potentiel de surface inverse dans le récipient, de préférence en utilisant une pompe à seringue de 50 à 500 µl h-1, par exemple 100 à 300 µl h-1, idéalement environ 200 µl h-1, afin d'obtenir un mélange de cellules, et
- le mélange de la mixtion de cellules pendant 5 à 60 minutes, par exemple 10 à 40 minutes, idéalement environ 20 min.

2. Procédé selon la revendication 1, où la seconde série de cellules est composée de cellules naturelles sans surface modifiée, ou bien les cellules ont une surface modifiée avec un potentiel de surface négatif et ont été obtenues par un procédé comprenant les étapes de :
i. la culture de cellules dans un milieu de croissance approprié jusqu'à l'OD approprié, les cellules étant lavées et facultativement traitées pour briser d'éventuelles structures multicellulaires causées par la floculation spontanée, la coagulation ou l'agrégation de cellules,
ii. le traitement des cellules avec une solution de polyélectrolyte de charge positive et de 0,025 à 0,25% p/v ;
iii. le retrait du polyélectrolyte en excès, puis
iv. le traitement des cellules obtenues avec une solution de polyélectrolyte de charge négative et de 0,025 à 0,25% p/v, pour former la seconde série de cellules à surface modifiée
v. et enfin le retrait du polyélectrolyte de charge négative en excès.

3. Procédé selon la revendication 1 ou 2, dans lequel la première série de cellules obtenues à l'étape a) est en outre traitée avec une solution de polyélectrolyte de charge négative et de 0,025 à 0,25% p/v, et ensuite avec une solution de polyélectrolyte de charge positive et de 0,025 à 0,25 % p/v, où le nombre d'étapes de traitement supplémentaires est arbitraire.

4. Procédé selon l'une quelconque des revendications 1 à 3, dans lequel les cellules premières ou les cellules secondes à surface modifiée avant l'étape b) ont plusieurs couches de revêtement de polyélectrolyte, par exemple une première couche de polyélectrolyte de charge positive, une seconde couche de polyélectrolyte de charge négative, une troisième couche de polyélectrolyte de charge positive et des couches supplémentaires facultatives.

5. Procédé selon l'une quelconque des revendications 1 à 4, dans lequel le processus d'agrégation est contrôlé par l'ajustement de la concentration des cellules de noyau et d'enveloppe, le débit d'écoulement des cellules qui sont ajoutées dans la suspension de cellules de charges opposées, la vitesse de mélange/brassage, la quantité de cellules de charge opposée ajoutées à la suspension de cellules, et la force ionique de la suspension de cellules.

6. Procédé selon l'une quelconque des revendications 1 à 5, dans lequel la proportion des cellules de noyau par rapport aux cellules d'enveloppe est de 1:8 à 1,4:100.

7. Procédé selon l'une quelconque des revendications 1 à 6, dans lequel la première série de cellules à surface modifiée ou la seconde série de cellules est traitée avec des particules magnétiques afin de revêtir leur surface de ces particules ou d'incruster ces particules sur leur surface afin de permettre la séparation et l'enrichissement éventuel des agrégats formés, où le traitement avec des particules magnétiques est réalisé avant l'étape de mélange de la première série de cellules à surface modifiée avec la seconde série de cellules.

8. Procédé selon la revendication 7, dans lequel les particules magnétiques sont paramagnétiques.

9. Procédé selon la revendication 8, dans lequel les particules magnétiques sont des particules Fe₃O₄.

10. Procédé selon l'une quelconque des revendications précédentes, dans lequel les agrégats obtenus à l'étape b) sont en outre traités avec la première série de cellules ayant un potentiel de surface positif et faisant office de cellules troisièmes, pour former des amas de cellules premières recouvertes d'une couche de cellules secondes également recouvertes d'une couche de cellules troisièmes, les amas ainsi obtenus ayant un potentiel de surface positif, ou bien des agrégats composés de cellules secondes recouvertes d'une couche de cellules premières sont traités avec la seconde série de cellules ayant un potentiel de surface négatif, de façon à ce que les agrégats obtenus aient un potentiel de surface négatif, où le nombre de couches supplémentaires de cellules aux potentiels de surface alternés est arbitraire.

11. Procédé selon l'une quelconque des revendications précédentes, dans lequel chaque polyélectrolyte de charge positive est individuellement sélectionné parmi le polyéthylèneimine, le chlorure de polydiallyldiméthylammonium, le poly(hydrochlorure d'allylamine), le chitosane, l'amidon cationique et la cellulose aminée.

12. Procédé selon l'une quelconque des revendications précédentes, dans lequel chaque polyélectrolyte de charge négative est individuellement sélectionné parmi le polystyrène sulfonate, l'acide polyacrylique, l'alginate ou la cellulose.

13. Procédé selon l'une quelconque des revendications précédentes, dans lequel la première série de cellules, la seconde série de cellules et la troisième ou toute série supplémentaire de cellules sont de types différents.

14. Procédé selon l'une quelconque des revendications précédentes, dans lequel chaque cellule est une cellule microbienne, de préférence une cellule bactérienne.
